# EUROPEAN PATENT APPLICATION

(11) **EP 2 008 691 A1**
(43) Date of publication of application: **31.12.2008**
(21) Application number: 07012809.5
(22) Date of filing: 29.06.2007
(51) Int. Cl.: A61P 15/08, A61K 31/00

(54) **Vaginal odorants**

(71) Applicant: Fraunhofer-Gesellschaft zur Förderung der angewandten Forschung e.V., 80686 München (DE)
(72) Inventor: Büttner, Andrea, 81825 München (DE)
(74) Representative: Lux, Berthold

(57) **Abstract**

Use vaginal odorant compounds for controlling motile cell chemotaxis.

## Description

The present invention relates to the use of vaginal odorant compounds for controlling motile cell chemotaxis as well as to products comprising said vaginal odorant compounds.

Vaginal secretions represent a complex medium with variable composition due to diurnal and other physiological changes. The vaginal environment and its secretions has been described both in scientific as well as non-scientific literature as olfactorily active, and has been previously assumed to play a chemosensory or chemoattractive role in human communication and sexual attraction (Doty RL. Chem. Senses 1981, 6, 351-376). In addition, recent studies show that sperm chemotaxis is modulated by low-molecular, odor-active substances, and that odorant receptors are present on spermatozoae (Spehr M, Gisselmann G, Poplawski A, Riffell JA, Wetzel CH, Zimmer RK, Hatt H. Science 2003, 299, 2054-2058; Spehr M, Schwane K, Riffell JA, Barbour J, Zimmer RK, Neuhaus EM, Hatt H. J. Biol. Chem. 2004, 279, 40194-40203; Hatt H. DE 102 46 329 A1). A series of substances containing an aromatic (phenyl) moiety have been identified as agonists for these receptors as well as sperm-chemotaxis, such as bourgeonal, lilial, cyclamal, floralazone and several others. The response was found to be structure- and concentration-dependent, and was modified by the presence of additional low-molecular weight substances which also represent odor-active compounds, and act as antagonists for the observed effects. These were long-chained aliphatic aldehydes such as n-undecanal. Interestingly, the compounds described to date as the most active agonists/antagonists in sperm chemotaxis are not common natural odorants, but are predominantly used in synthetic washing and cleaning products, and in household applications. Apart from that, they are not among the common odor-active volatiles found in nature or foods, but exhibit relatively high odor-thresholds when compared to other key odorants e.g. from foods.

On the other hand, little is known about the natural odor substances occurring in the female sexual organs such as uterus, vagina. There is only some literature available on odor nuisances going along with e.g. vaginal infections such as bacterial vaginosis, or with menstrual or catamenial fluids (Wolrath H, Boren H, Hallen A, Forsum U. APMIS 2002, 110, 819-824; Wolrath H, Stahlbom B, Hallen A, Forsum U. APMIS 2005, 113, 513-516). Fishy and offensive odors have been described as being related to elevated amounts of trimethylamine of bacterial origin (Wolrath H, Boren H, Hallen A, Forsum U. APMIS 2002, 110, 819-824; Wolrath H, Stahlbom B, Hallen A, Forsum U. APMIS 2005, 113, 513-516; Brand JM, Galask RP. Obstetrics & Gynecology (New York, NY, United States) 1986, 68, 682-685). In addition, oxidation products such as trimethylamine oxide were found. Another study reported caproic acid in relation to a foul vaginal odor (Stanek R, Glover DD, Mya S, Larsen B. Biomed. Chrom. 1987, 2, 76-78). Apart from that, several vaginal organic acids such as acetic and other short-chain aliphatic acids and lactic acid have been described, some of them being postulated as human pheromones, but also as vaginal compounds in monkeys such as chimpanzees (Bauman JE, Kolodny RC, Webster SK. Fertil Steril. 1982, 38, 572-579; Waltman R, Tricomi V, Wilson GE Jr, Lewin AH, Goldberg NL, Chang MM. Lancet 1973, 2, 496; Fox GJ. Folia Primatol (Basel). 1982, ;37, 255-266.). In this context, these acid substances were termed as "copulines" (Michael RP, Bonsall RW, Kutner M. Psychoneuroendocrinology. 1975, 1, 153-163). The authors reported that acetic acid is found in human vaginal fluids, and that propanoic, methylpropanoic, butanoic, isovaleric, and 2-methylpentanoic acid are found en bloc in about one-third of women tested, but not in the other two-thirds. Among the women who had all "copulins", the quantities of these substances increased during the fertile pre-ovulatory week of the menstrual cycle, and dropped sharply after ovulation. Another study confirmed the production of copulins in one third of the women, but did not confirm the monthly cycle of copulin production (Huggins GR, Preti G. Clin. Obstet. Gynecol. 1981, 24, 355-377).

Generally, vaginal odors, predominantly malodors, have been analysed by means of high performance ion exchange chromatography, direct headspace-gas chromatography as well as other gas chromatographic techniques after collection of vaginal secretions by means of wipes, sponges or tissues. In several studies, complex workup procedures have been applied such as solvent extraction, pH changes or diverse chromatographic steps. Chemical identity was confimed by means of mass spectral data. However, identification of the key components was based on dominance of certain peaks as they appear in the chromatographic separation but not on olfactory potency as it is done for example in gas chromatograohy olfactometry. Therefore, a rating of the real contribution to the described odor phenomena is not possible in these studies, as it remained unclear whather there wer additional trace odor-active substances with even higher odor impact. Only very early works from Dravnieks et al. involved gas chromatographic olfactometric techniques for characterization of odorants that had been isolated before by a type of intra-vaginal gas stripping technique comparable to conventional purge and trap headspace analysis, only that it had been performed intra-vaginally using an in-vivo gas stripping device (Dravnieks A, Krotoszynski BK, Keith L, Bush IM. J. Pharm. Sci. 1970, 59, 459-501). While a series of compounds could be separated and described by means of olfactometry, authors did not succeed to identify any substances. In another study, an experiment was conducted with three women to determine the odor composition of vaginal secretions before and after coitus (Keith L, Draunieks A, Krotoszynski BK. Arch Gynakol. 1975, 218, 203-204). A GC/sensory assay (odorogram) was used and odor description notes were made, indicating that thirteen odorous compounds occurred regularly. It was noted that components with acidic odor tended to appear at lower retention rates in postcoital samples. However, the chemistry of these compounds was not investigated. The authors concluded that differences exist in the odors of pre- and postcoital vaginal secretions, but the biological relevance of these differences remained unresolved, as well as the question of the pheromonic significance of vaginal odors in humans.

Apart from antimicrobial treatment of these infections (US 2005/186255), olfactory control or covering with other odor-active substances such as musks or salicylates has been described, being administered for example as sanitary pads (WO 2004/098666).

However, nothing is known about the ability of the female follicle to produce odor-active substances, or about the odor composition of the follicle and the follicular fluid, respectively.

Thus the object of the present invention is to find a new way to control the movment of motile cells, in particular sperms. Another object of the present invention is enhance the sexual stimulation of human and/or not human beings. It is in particular an object of the present invention to enhance male and/or female fertility. Additionally it is sought for articles being contraceptive or being fertility supporting. Finally it is sought for a new diagnostic test to determine the activity of motile cells, like sperms.

The first finding of the present invention is that the vaginal odorant compounds fulfill the objects of the instant invention. The second finding of the present invention is that an analytical approach is needed that allows the unambiguous identification of odor-active trace compounds intra-vaginaly or from collected small-scale vaginal secretions. This approach should further offer the possibility to analyse vaginal secretions from individual donors in a non-invasive set-up with minimum disturbance of the natural vaginal environment, and without the need to pool samples from a number of donors. Also, the technique should allow the analysis of vaginal secretions with high temporal resolution, to monitor day-to-day or diurnal variations. The technique should be also applicable for characterization of volatile organic substances and odor-active substances in menstrual/catamenial fluids, as well as in follicular and seminal fluid.

Thus the present invention is directed to the use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin for controlling motile cell chemotaxis.

The above listed vaginal odorant compounds can be used alone or in combination with each other and/or in combination with other odorant compounds, preferably other vaginal odorant compounds, like n-octanal, acetic acid, butanoic acid, 2/3 methylbutanoic acid or phenylacetaldehyde. It is in particular preferred that at least two, like two, three or four, vaginal odorant compounds of the above list are used together.

It has been found out that the above compounds are vaginal odorant compounds, i.e. are part of the vaginal secretion. Moreover the above listed vaginal odorant compounds are potent substances which positively influences the activity and/or the direction of movement of the motile cells, like sperms. Some of the vaginal odorant compounds are potential attractants whereas others are disincentives.

Accordingly the vaginal odorant compound selected from the group consisting of hydrogen sulfide, methylpropanal, butan-2,3-dione (diacetyl), 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin are activating for motile cells, like sperms, i.e. the motile cells, like sperms, are activated to move in the direction of said compounds. Preferred vaginal odorant compounds of said list are those selected from the group consisting of 1-octen-3-one, 2-acetyl-1-pyrroline, 2-acetylpyridine, 2-acetylthiazoline, 2-acetyltetrahydropyridine, 2-acetylpyrazine, (E)-β-damascenone, β-damascone, β-ionone and α-ionone, more preferably selected from the group consisting (E)-β-damascenone, β-damascone, β-ionone and α-ionone and yet more preferably the vaginal odorant compound is (E)-β-damascenone and/or β-ionone. To improve the movement of the motile cells, preferably sperms, at least two, like two, three or four, vaginal compounds of the lists as stated in this paragraph are used together. It is in particular preferred, that (E)-β-damascenone and β-ionone are used together. Of course the above listed vaginal odorant compounds can be used in combination with other odorant compounds, preferably other vaginal odorant compounds, like acetic acid, butanoic acid, 2/3 methylbutanoic acid or phenylacetaldehyde. Thus the vaginal odorant compounds as described in this paragraph are suited to direct the motile cells, like sperms, in a specific direction, for instance in the direction to an ovum, i.e. to a follicle. The ovum can be ex vivo or in vivo, i.e. in the vagina of a female, located.

Accordingly the vaginal odorant compound as stated in the previous paragraph are used to improve the fertility of males and/or females, in particular of human beings but also of animals, like domestic animals, e.g. cattle, swine or sheep. The subjects to be treated include healthy subjects or subjects with a reduced fertility. In particular the vaginal odorant compounds of the previous paragraph are used to improve the fertility of males and/or females having reduced fertility. Men for instance might produce only or particularly sperms with less receptor activity for vaginal odorant compounds and/or sperms with reduced amount of receptors sensitive to vaginal odorant compounds compared to normal sperms. Accordingly an enhanced concentration of vaginal odorant compounds in the vagina is needed to direct the sperms to the follicle, i.e. the ovum. On the other hand, females might produce to little vaginal odorant compound concentration so that the sperms are not guided sufficiently to the follicle, i.e. to the ovum. Also in this case the concentration of the vaginal odorant compounds in the vagina must be increased to improve the fertilization of the female. Therefore in a further aspect of the present invention the vaginal odorant compounds as sated in the previous paragraph are used to increase the concentration of the vaginal odorant compounds. In particular those compounds are used which especially improves the activity of motile cells, like sperms. Preferred representatives of especially good vaginal odorant compounds are those selected from the group consisting of 1-octen-3-one, 2-acetyl-1-pyrroline, 2-acetylpyridine, 2-acetylthiazoline, 2-acetyltetrahydropyridine, 2-acetylpyrazine, (E)-β-damascenone, β-damascone, β-ionone and α-ionone, more preferably selected from the group consisting (E)-β-damascenone, β-damascone, β-ionone and α-ionone and yet more preferably the vaginal odorant compound is(are) (E)-β-damascenone and/or β-ionone. At least two, like two, three or four, vaginal odorant compounds of the lists as stated in this paragraph are used together. The vaginal odorant compounds are in particular used in the vagina, i.e. close to the uterus or follicle. Thus the vaginal odorant compounds as specified in this and previous paragraph are used in a article selected from the group consisting of vaginal crème, vaginal suppository, vaginal irrigation, vaginal lotion, vaginal ointment, tampon and sanitary napkin.

A further aspect of the present invention is therefore the use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin for the manufacture of a pharmaceutical composition for treatment of reduced fertility of males and/or females, in particular for the treatment of reduced fertility of males and/ore females caused by the defects mentioned above, i.e. by reduced receptor activity of sperms for vaginal odorant compounds and/or reduced amount of receptors on sperms sensitive to vaginal odorant compounds and/or reduced vaginal odorant compound concentration in females.

Of course the present invention is also directed to articles that improve fertility. Thus the present invention is directed to vaginal articles, in particular articles selected from the group consisting of vaginal crème, vaginal suppository, vaginal irrigation, vaginal lotion, vaginal ointment, tampon and sanitary napkin, wherein said articles comprise at least one vaginal odorant compound selected from the group consisting of hydrogen sulfide, methylpropanal, butan-2,3-dione (diacetyl), 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, 5-dodecalactone, 3-methylindole (skatol), indole, 2-aminoacetophenone, phenylacetic acid and vanillin. Preferably the article comprises at least one vaginal odorant compound selected from the group consisting of 1-octen-3-one, 2-acetyl-1-pyrroline, 2-acetylpyridine, 2-acetylthiazoline, 2-acetyltetrahydropyridine, 2-acetylpyrazine, (E)-β-damascenone, β-damascone, β-ionone and α-ionone, more preferably selected from the group consisting (E)-β-damascenone, β-damascone, β-ionone and α-ionone, yet more preferably the vaginal odorant compound is(are) (E)-β-damascenone and/or β-ionone. To improve the fertility of male and/or female the vaginal odorant compounds as listed in this paragraph can be present in the article alone or in combination with each other and/or in combination with other odorant compounds, preferably other vaginal odorant compounds, like acetic acid, butanoic acid, 2/3 methylbutanoic acid or phenylacetaldehyde. It is in particular preferred that at least two, like two, three or four, vaginal odorant compounds of the above list are comprised in the article. In another preferred embodiment acetic acid, butanoic acid, 2/3 methylbutanoic acid and/or phenylacetaldehyde is/are used together with at least one vaginal odorant compound selected from the group consisting of 1-octen-3-one, 2-acetyl-1-pyrroline, 2-acetylpyridine, 2-acetylthiazoline, 2-acetyltetrahydropyridine, 2-acetylpyrazine, (E)-β-damascenone, β-damascone, β-ionone and α-ionone, more preferably selected from the group consisting (E)-β-damascenone, β-damascone, β-ionone and α-ionone, yet more preferably the vaginal odorant compound is(are) (E)-β-damascenone and/or β-ionone.

Preferably the articles comprises further additives in particular further compounds additionally improving the fertility. The term "vaginal article" as used throughout the instant invention expresses that the article must be essentially free of impurities, i.e. the article must fulfill the requirements of vaginal products. Such products must for instance compliant with the mucosa. The vaginal compositions may maintain the vaginal pH value, meaning that the compositions have a pH of 5 or below, preferably from 3.5 to 4.5.

On the other hand, as stated above, the vaginal odorant compounds may be used to lead the motile cells, like sperms, astray, i.e. away from the follicle, i.e. from the ovum. In other words the vaginal odorant compounds as defined in the instant invention may be used as a contraceptive. Accordingly the present invention is in particular directed to the use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin as a contraceptive, more preferably for the manufacture of a contraceptive. Preferred representatives of especially good vaginal odorant compounds for this purpose are those selected from the group consisting of n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, (E)-β-damascenone, β-damascone, β-ionone and α-ionone. In particular preferred is the use of at least one compound selected from n-hexanal, n-heptanal, n-nonanal. It is in particular preferred that at least two, like two, three or four, vaginal compounds of the lists as stated in this paragraph are used together. To improve the contraceptive effect the vaginal odorant compounds as listed in this paragraph can be used alone or in combination with each other and/or in combination with other odorant compounds, preferably other vaginal odorant compounds, like n-octanal. It is in particular preferred that at least two, like two or three, vaginal odorant compounds of the above list are used together. In another preferred embodiment n-octanal is used together with at least one vaginal odorant compound selected from the group consisting of n-hexanal, n-heptanal and n-nonanal. It is in particular appreciated that together with the vaginal odorant compounds of this paragraph spermicide compounds are used. Thus the present invention is also directed to the use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin, more preferably selected from the group consisting of n-hexanal, n-heptanal and n-nonanal, still more preferably together with spermicide compounds, as a contraceptive in a article selected from the group consisting of vaginal crème, vaginal suppository, vaginal irrigation, vaginal lotion, vaginal ointment, tampon and sanitary napkin. Preferably the vaginal odorant compounds as defined in this paragraph are used as a contraceptive in non private area. Accordingly the vaginal odorant compounds as defined in this paragraph are used as a contraceptive for non-human beings, preferably for animals, like domestic animals, e.g. cattle, swine or sheep.

Of course the present invention is also directed to vaginal articles that are contraceptives. Thus the present invention is directed to vaginal articles selected from the group consisting of vaginal crème, vaginal suppository, vaginal irrigation, vaginal lotion, vaginal ointment, tampon and sanitary napkin, wherein said articles comprise
(a) at least one vaginal odorant selected from hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin, preferably at least one vaginal odorant compound selected from the group consisting of n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, (E)-β-damascenone, β-damascone, β-ionone and α-ionone, more preferably at least one vaginal odorant compound selected from the group consisting of n-hexanal, n-heptanal and n-nonanal,
(b) optionally spermicide compounds.

To improve the contraceptive effect of the articles the vaginal odorant compounds as listed in the previous paragraph can be used alone or in combination with each other and/or in combination with other odorant compounds, preferably other vaginal odorant compounds, like n-octanal. It is in particular preferred that at least two, like two or three, vaginal odorant compounds of the above list are present in the vaginal articles. In another preferred embodiment the articles comprise n-octanal and at least one vaginal odorant compound selected from the lists as stated in the paragraph above. It is in particular appreciated that the articles comprise additionally spermicide compounds. The vaginal articles may maintain the vaginal pH value, meaning that the compositions have a pH of 5 or below, preferably from 3.5 to 4.5.

As the vaginal odorant compounds of the present invention have also a therapeutic effect, for instance to improve fertility, the invention is also directed to the first medical use of said compounds. Thus the instant invention is directed to a vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, vanillin and mixtures thereof as a medicament. More preferably the instant invention provides a vaginal odorant compound selected from the group consisting of hydrogen sulfide, methylpropanal, butan-2,3-dione (diacetyl), 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, vanillin and mixtures thereof as a medicament. It is in particular preferred that at least two, like two, three or four, vaginal compounds of the lists as stated in this paragraph are used together.

Another aspect in which the vaginal odorant compounds of the present invention can be applied is the technical field of diagnostic tests. Vaginal odorant compounds are suitable to evaluate whether motile cells, like sperms, have normal chemotaxis, for instance in the direction of follicle, i.e. in the direction of the ovum, which is surrounded by said vaginal odorant compounds. Thus the present invention is also directed to the use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin or mixtures thereof for testing whether the chemotaxis of motile celles, like sperms, is disordered, i.e. reduced or totally lapsed, in particular with regard to said compounds. In a further embodiment at least two, like two, three or four, vaginal compounds of the list as stated in this paragraph are used together. In other words it can be tested whether the receptor activity of the sperms for vaginal odorant compounds is abnormal and/or the amount of receptors on sperms sensitive to vaginal odorant compounds is reduced or elevated compared to the standard. Preferably the diagnostic test is not practised on the human or animal body.

Of course the instant invention is also directed to the diagonostic test as such. Accordingly the present invention provides a diagnostic test comprising at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin. In a further embodiment at least two, like two, three or four, vaginal compounds of the list as stated in this paragraph are used together.

The vaginal odorant compounds of the present invention are not only suitable to influence the chemtaxis of motile cells, like sperms, but also suitable to mimic or imitate vaginal smell. Thus the present invention is also directed to the use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, acetic acid, butanoic acid, 2/3 methylbutanoic, phenylacetaldehyde and vanillin for mimicking or imitating vaginal smell. It is however preferred to use at least one vaginal odorant compound selected from the group consisting of hydrogen sulfide, methylpropanal, butan-2,3-dione (diacetyl), 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid and vanillin for mimicking or imitating vaginal smell.

The listed vaginal odorant compounds can be used for this purpose alone or in combination with each other. It is in particular preferred that at least two, like two, three or four, vaginal odorant compounds of the above list are used together to obtain an especially intensive vaginal smell. The vaginal odorant compounds and the combinations thereof are in particular used as a sexual stimulant, like for drinks or oils, like cutaneous tolerant oils, fluids, like cutaneous tolerant fluids, or cremes, like cutaneous tolerant cremes, or used on fetish articles, like toys, but also on cloth, like pants or underwear, pillows and blankets.

Accordingly the present invention encompasses fetish articles, toys, cloths, like pants or underwear, pillows, blankets, sexual stimulant, like drinks, oils, especially cutaneous tolerant oils, fluids, especially cutaneous tolerant fluids, or cremes, especially cutaneous tolerant crèmes, that comprise at least vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, vanillin, n-octanal, acetic acid, butanoic acid, 2/3 methylbutanoic acid, phenylacetaldehyde and mixtures thereof. It is however preferred that fetish articles, toys, cloths, like pants or underwear, pillows, blankets, sexual stimulants, like drinks, oils, especially cutaneous tolerant oils, fluids, especially cutaneous tolerant fluids, or cremes, especially cutaneous tolerant crèmes, comprise at least vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, vanillin and mixtures thereof. It is in particular preferred that at least two, like two, three or four, vaginal compounds of the list as stated in this paragraph are used together.

In order to find the above described vaginal odorant compounds the present inventor has studied the odor composition of fresh human vaginal secretion and the intra-vagenal odor environment by appropriate analytical tools without the need of intermittent sampling and sample workup steps, without any preseparation or other common preconcentration steps, so that artefact formation is maintained at minimum. For analysis and characterization predominantly the aspect of odor-activity was taken into account.

The analytical approach comprising gas chromatographic-olfactometric characterization of the odor volatiles was used that allowed the unambiguous identification of odor-active compounds even in small-scale human vaginal odor samples.

The present invention shows that the technique can be used both for *in vivo* direct extractive sampling of odorants from vaginal secretions, and for analysis of *ex vivo* sampled vaginal secretions. The methodology was successfully applied for identification of more than twenty characteristic odorants in the vaginal environment and vaginal secretion, respectively. Due to its convenient and non-invasive applicability, even day-to-day or diurnal physiological variations in the profiles of volatile organic compounds in vaginal secretions can be monitored.

In the following the present invention is further described by way of examples.

### Examples

### Chemicals

The following reference compounds were obtained from the suppliers shown: 2'-aminoacetophenone ≥98 %, 5a-androst- 16-en-3α-one ≥98 %, butane-2,3-dione ≥99 %, butanoic acid ≥99.5 %, (*E,E*)-deca-2,4-dienal 85 %, (+/-)-δ-decalactone ≥98 %, (+/-)-γ-decalactone ≥98 %, decanal ≥99 %, (+/-)-δ -dodecalactone ≥98 %, (Z)-6-γ-dodecenolactone ≥85 %, 4-hydroxy-2,5-dimethyl-3(2*H*)-furanone ≥99 %, 3-hydroxy-4,5-dimethyl-2(5*H*)-furanone (sotolon) ≥987%, hexanal 98 %, 3-isobutyl-2-methoxypyrazine ≥99 %, (+/-)-linalool ≥97 %, 3-methylindole (skatol) ≥99 %, methylpropanal ≥99 %, 3-(methylthio)-propanal 85 %, (+/-)-2- and 3-methylbutanoic acid 99 %, (*E,E*)-2,4-nonadienal ≥85 %, (*E*/*Z*)-2,6-nonadienal 95 %, (+/-)-γ-nonalactone ≥98 %, nonanal ≥95 %, (*E*)-2-nonenal 97 %, (*E,E*)-octa-2,4-dienal ≥96 %, octanal 99 %, (*E*)-oct-2-enal ≥94 %, pentanoic acid ≥99 %, phenylacetaldehyde ≥90 %, phenylacetic acid ≥99 %, 2-phenylethanol ≥99 % (Sigma-Aldrich, Steinheim, Germany), acetic acid ≥99 %, 2-methoxyphenol ≥99.5 %, vanillin ≥99 % (Merck, Darmstadt, Germany), (*E*)-β-damascenone 98 % (Haarmann and Reimer, Holzminden, Germany), β -ionone ≥99 % (Roth, Karlsruhe, Germany), 1-octen-3-one 95 % (Lancaster, Mühlheim, Germany). The following compounds were synthesized according to the literature given in brackets: *tr*-4,5-Epoxy-(*E*)-2-decenal (Schieberle P, Grosch W. Z. Lebensm. Unters. Forsch. 1991, 192: 130-135),1-hexen-3-one (Blank I, Fischer KH, Grosch W. Z. Lebensm. Unters. Fosch. 1989, 189: 426-433), (Z)-octa-1,5-dien-3-one (Ullrich F, Grosch W. J. Am. Oil Chem. Soc. 1988, 65: 1313-1317), (Z)-non-2-enal (Ullrich F, Grosch W. Fat Sci. Technol. 1988, 90: 332-336), 4-ethyloctanoic acid (Rota V. Charakterisierung von Schlüsselaromastoffen in rohem und gekochtem Schaffleisch durch Anwendung von Struktur/Wirkungskonzepten. PhD thesis, Technical University Munich, Germany, Verlag Dr. Hut, Muenchen, Germany, 2004). The compounds were freshly distilled prior to analysis. Chemical and sensory purity was checked by gas chromatography-olfactometry (GC/O) as well as gas chromatography-mass spectrometry (GC-MS).

### Samples

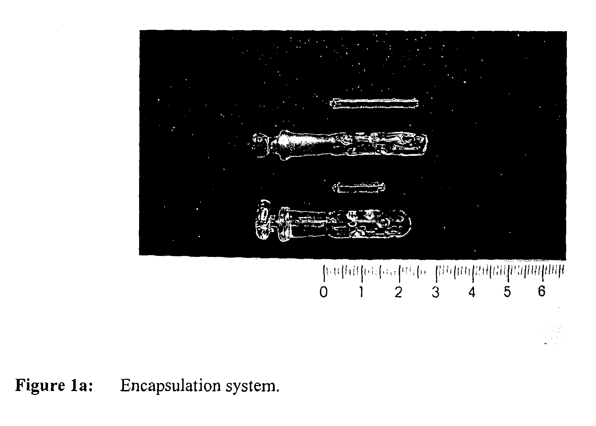

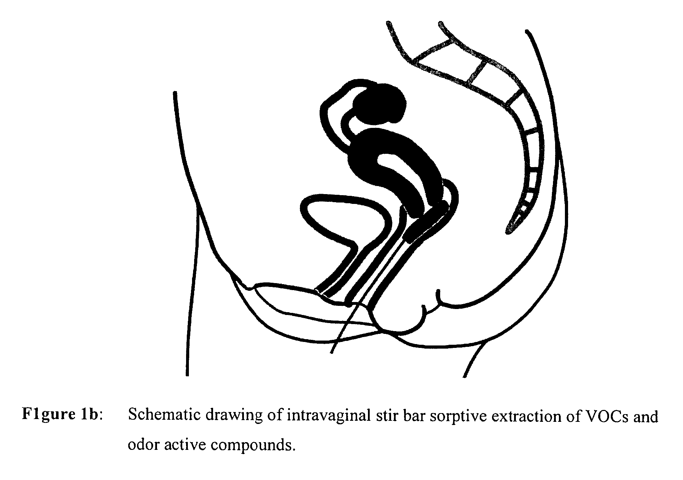

Twenty intra-vaginal sampling episodes were conducted for three different donors. The intra-vaginal sampling was achieved using an encapsulated SBSE stir bar (cf. Figure 1a and b). Panelists were non-pregnant volunteers (non-smokers, Germans of caucasian ethnicity) in the age range 28-35 (mean age 32), exhibiting no known illnesses or genital infections at the time of examination. Prior to sample collection and analysis, written consent has been obtained from all participants participating in the study after full explanation of the purpose and nature of the study.

### PDMS-coated stir bars

For the experiments, commercially available Twister®-SBSE bars (20 mm length, 0.5 mm PDMS coating thickness and ~ 50 µL of total PDMS volume according to the suppliers specifications; Gerstel GmbH, Mühlheim a/d Ruhr, Germany) were used. Prior to analysis, the bars were subjected to a conditioning procedure according to the suppliers recommendations: the stir bars were first soaked in 100 % acetonitrile for at least two days, then conditioned at 300 °C for 4h.

Each SBSE bar was first screened for odorants ("background", see "Results and Discussion") and then directly used for analysis. Each stir bar was used for just one single experiment, then reconditioned and screened for background again. Each experiment was performed with at least three different SBSE bars to avoid SBSE bar variations.

### Encapsulation of the SBSE bars

For intra-oral application, adapted glass capsules were designed (cf. Fig. 1 a). For the 20 mm bars, the total length of the capsule was 25 mm, for the 10 mm bar 15 mm. The inner diameter was in both cases 5 mm. The capsules were sealed with a glass stopper. To allow unhindered penetration of air and saliva, the capsules were regularly perforated with pores (1-2 mm diameter) with a distance of about 3 mm between pores.

### Direct Intra-Vaginal Stir Bar Sorptive Extraction of Volatile Organic Compounds

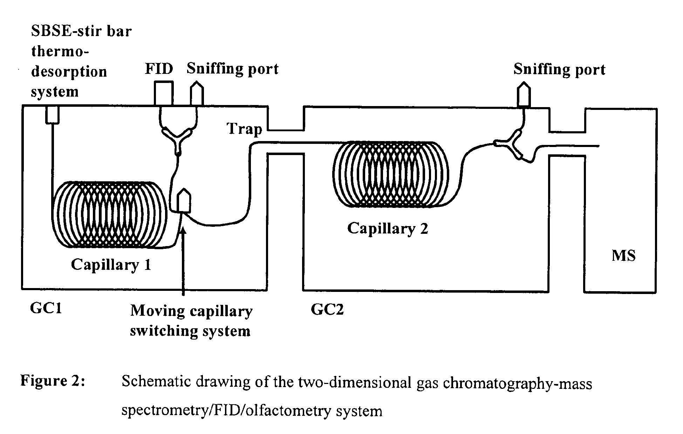

Prior to analysis, the capsule system was sterilized by heat treatment in boiling distilled water for 15 min. Then, a freshly conditioned and background-screened SBSE bar (see above) was encapsulated and immediately placed intra-vaginally as displayed in Figure 1b, and equilibrated for 15 min. After incubation, the capsule was removed, the SBSE bar was recovered from the capsule with tweezers, dipped into deodorized water, briefly dried with lint-free tissue and immediately placed into the thermo-desorption unit. The analytical setup of the two-dimensional GC-MS/Olfactometry/FID/MS system is displayed in Figure 2.

### Ex-Vivo Pad SBSE-Sampling

A freshly conditioned and background-screened SBSE bar (see above) was immediately placed into a small liquid absorbing cotton-fabricated pad that was used then in the style of a sanitary pad, and equilibrated for 15, 30 or 60 min, respectively. After incubation, the capsule was removed from the pad, the SBSE bar was recovered from the capsule with tweezers, dipped into deodorized water, briefly dried with lint-free tissue and immediately placed into the thermo-desorption unit. The analytical setup of the two-dimensional GC-MS/Olfactometry/FID/MS system is displayed in Figure 2.

### SBSE Thermo-Desorptive Sample Application

Thermo-desorption of the samples was performed by means of a TDS-2 thermo-desorption system (Gerstel GmbH) in combination with a CIS-4 PTV injector (Gerstel GmbH) for cryo focussing the analytes prior to transfer onto the analytical column. The following sampling parameters were used: Splitless thermal desorption was performed by programming the TDS-2 from 40 °C to 240 °C (5 min) at a rate of 60 °C. Cryo focussing was performed with liquid nitrogen at -100 °C. Injection was performed with a ramp of 12 °C/s from -100 °C to 240 °C (5 min). The gas chromatographic conditions are given below.

### High Resolution Gas Chromatography-Olfactometry

Application of the samples was performed as described above *(SBSE Thermo-Desorptive Sample Application).* The odorants were screened in parallel by three panelists by sniffing the effluent after gas chromatographic separation. Sniffing analysis was repeated three times by each panelist. All detected odorants were identified by comparison with reference substances on the basis of the following criteria: retention index (RI) on two stationary phases of different polarity (DB-FFAP, DB-5), mass spectra obtained by MS (EI) and MS (CI), and odor quality as well as odor intensity perceived at the sniffing-port. Only if the odor quality and intensity of the reference agrees with that detected via GC-O, identification can be regarded as "positive".

The one- (for sniffing) or two-dimensional (for mass spectrometric identification) gas chromatographic system (TD-HRGC) consisted of a Mega 2 gas chromatograph (Fisons Instruments, Mainz-Kastel, Germany) as the precolumn system in tandem with a Fisons GC 5160 as the main column system (cf. Figure 2). The following fused silica columns were used: DB-FFAP (30 m x 0.32 mm i.d., 0.25 µm FD, J & W Scientific, Folsom, USA) and/or DB-5 (SE-54; 30 m x 0.32 mm i.d., 0.25 µm FD, J & W Scientific, Folsom, USA). The gas chromatographic conditions were the same as described previously (Buettner A, Schieberle P. J. Agric. Food Chem. 2001, 49: 1358-1363).

### Rating of Odorants using the Vaginal Odor Screening (VOS)-System

Detectability of the odorants was based on their sensory properties, that means first and foremost on their odor intensities. That means that only those substances were rated as detectable by the Vaginal Odor Screening System (VOSS) which were perceived by HRGC/O. Detection by HRGC/MS or HRGC/FID was not taken into account as this does not necessarily correlate with the sensory impact of the respective compound.

### High Resolution Gas Chromatography-Mass Spectrometry

The odorants were analyzed by two-dimensional gas chromatography (TD-HRGC) as described above. MS analyses were performed in parallels with the sniffing analysis on the main column system with an ITD-800 (Fisons Instruments, Mainz-Kastel, Germany) running in the CI-mode with methanol as the reagent gas. The following fused silica columns were used: DB-FFAP (30 m x 0.32 mm i.d., 0.25 µm FD, J & W Scientific, Folsom, USA) in combination with DB-5 (SE-54; 30 m x 0.32 mm i.d., 0.25 µm FD, J & W Scientific, Folsom, USA). The gas chromatographic and mass spectrometric conditions were the same as described previously (Buettner A, Schieberle P. J. Agric. Food Chem. 2001, 49: 1358-1363).

### RESULTS

### Characterization of odor-active compounds via intra-vaginal PDMS bar sampling

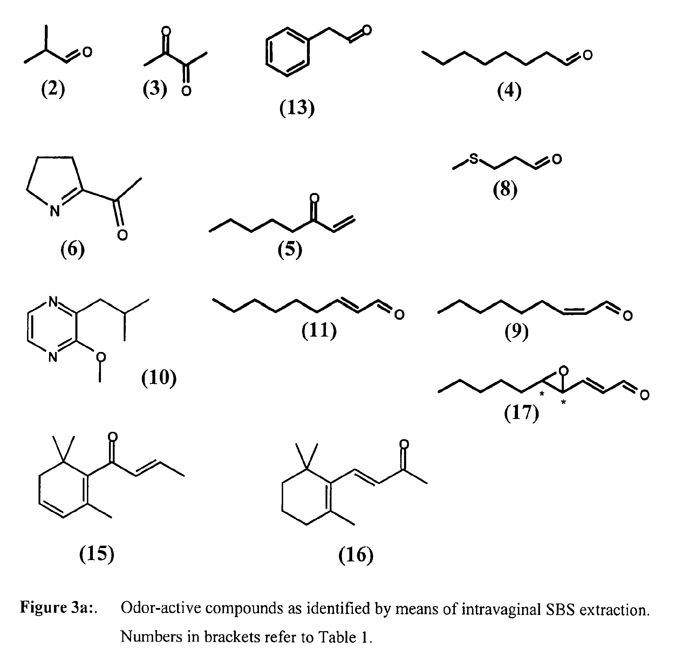

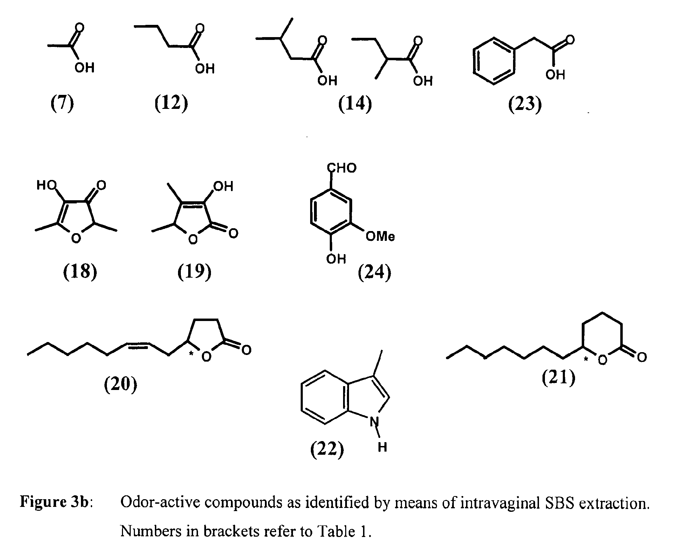

When analyzing the intra-vaginally adsorbed odorants by means of HRGC-olfactometry, a total of 26 odor-active compounds was detected. All of these were identified based on the criteria given in the experimental section (cf. Table 1). Among the detected compounds were flowery and fruity compounds such as β-damascenone with cooked apple smell, the peach-like smelling (Z)-6-γ-dodecenolactone, and the violet-like smelling β-ionone. Acidic and sweaty impressions were due to acetic acid, butanoic acid and the methylbutanoic acids. Apart from that, several sweet smelling substances were detectable, such as the honey-like smelling phenylacetic acid, phenylacetaldehyde, δ-dodecalactone, as well as 4-hydroxy-2,5-dimethyl-3(2*H*)-furanone (caramel-like), and vanillin (vanilla-like). Metallic, mushroom- and geranium leaf-like substances were oct-1-en-3-one, and *tr*-(4,5)-epoxy-(*E*)-2-decenal. Specific malty, buttery and cooked potato-like impressions were elicited by methylpropanal, diacetyl, and methional, respectively. Others were the savory-spicy-like smelling sotolone, the faeces-like smelling skatol, the sulfury hydrogen sulfide, the citrussy octanal, and the popcorn-like 2-acetylpyrroline, and Isobutyl methoxypyrazine with bellpepper-like smell. Fatty impressions came from the two nonenals. The chemical structures of these odorants are shown in Figures 3a and b.

**Table 1: Intra-vaginal characterization of odor-active compounds by means of SBSE-gas chromatography-olfactometry/mass spectrometry.**

| No. | Odorant ^{a} | Odor Quality ^{b} | Sampling technique ^{c} | Retention index^{e} on | |
|---|---|---|---|---|---|
| | | | | DB-FFAP | DB-5 |
| 1 | Hydrogen sulfide | sulfury | IV | | |
| 2 | Methylpropanal | malty | IV | 0821 | 0552 |
| 3 | Butan-2,3-dione (Diacetyl) | buttery | IV | 0981 | 0596 |
| 4 | Octanal | citrussy, soapy | IV | 1280 | 1004 |
| 5 | 1-Octen-3-one | mushroom-like | IV | 1295 | 0980 |
| 6 | 2-Acetyl-1-pyrroline | popcorn-like | IV | 1346 | 923 |
| 7 | Acetic acid | acidic | IV | 1451 | Nd |
| 8 | 3-Methylthio propanal (Methional) | cooked potato | IV | 1452 | 0905 |
| 9 | (Z)-Non-2-enal | fatty, tallowy | IV | 1502 | 1148 |
| 10 | 3-Isobutyl-2-methoxypyrazine | bellpepper-like | IV | 1517 | 1184 |
| 11 | (*E*)-Non-2-enal | fatty, cucumber-like | IV | 1527 | 1161 |
| 12 | Butanoic acid | sweaty | IV | 1619 | 0821 |
| 13 | Phenylacetaldehyde | honey-like | IV | 1642 | 1047 |
| 14 | 2/3-Methylbutanoic acid^{f)} | sweaty | IV | 1661 | 0875 |
| 15 | (*E*)-β-Damascenone | cooked apple-like | IV | 1807 | 1411 |
| 16 | β-Ionone | violet-like | IV | 1933 | 1491 |
| 17 | *tr*-(4,5)-Epoxy-(*E*)-dec-2-enal | metallic | IV | 2006 | 1382 |
| 18 | 4-Hydroxy-2,5-dimethyl-3(2*H*)-furanone | caramel-like | IV | 2031 | 1070 |
| 19 | 3-Hydroxy-4,5-dimethyl-2(5*H*)-furanone (Sotolon) | savory-like | IV | 2196 | 1110 |
| 20 | (Z)-6-γ-Dodecenolactone^{f)} | peach-like, green | IV | 2380 | 1670 |
| 21 | δ-Dodecalactone^{f)} | sweet | IV | 2426 | 1715 |
| 22 | 3-Methylindole (Skatol) | faeces-like | IV | 2484 | 1388 |
| 23 | Phenylacetic acid | honey-like | IV | 2551 | 1262 |
| 24 | Vanillin | vanilla-like | IV | 2569 | 1404 |

| | | | | | |
|---|---|---|---|---|---|
| ^{a} The compounds were identified by comparing it with the reference odorant based on the following criteria: retention indices on the capillaries named in the table, mass spectra obtained by MS/EI and MS/CI, odor quality and intensity perceived at the sniffing port. ^{b} Odor quality perceived at the sniffing port. ^{c} Sampling technique: HS: HRGC-Olfactometric detection of odorants via PDMS bar - headspace sampling, IV: HRGC-Olfactometric detection of odorants via direct intravaginal stir bar sorptive extraction. ^{e} Retention indices were calculated according to [xxx]. ^{f} The stereochemistry was not determined. | | | | | |

## Claims

1. Use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, and vanillin for controlling motile cell chemotaxis.

2. Use according to claim 1, wherein the motile cell is a sperm, in particular a sperm of non-human being.

3. Use according to claim 1 or 2, wherein the fertility, in particular the male fertility and/or female fertility, is(are) enhanced.

4. Use according to claim 3, wherein at least one vaginal odorant compound is selected from the group consisting of hydrogen sulfide, methylpropanal, butan-2;3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, (E)-β-damascenone, β-damascone, β-ionone, α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, and vanillin, preferably selected from the group consisting of (E)-β-damascenone, β-damascone, β-ionone and α-ionone.

5. Use according to claim 1 or 2 as a contraceptive.

6. Use according to claim 5, wherein at least one vaginal odorant compound is selected from the group consisting of n-hexanal, n-heptanal and n-nonanal.

7. Use according to claim 1 or 2 as a diagnostic test to determine the activity of a motile cell, in particular of a sperm.

8. Use according to claim 7, wherein the diagnostic test determines the receptor activity of the motile cell, in particular of the sperm.

9. Use of at least one vaginal odorant compound selected from the group consisting of hydrogen sulfide, methylpropanal, butan-2,3-dione (diacetyl), n-hexanal, n-heptanal, n-octanal, n-nonanal, 1-octen-3-one_{;} 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, acetic acid, butanoic acid, 2/3 methylbutanoic acid, phenylacetaldehyde and vanillin for mimicking or imitating vaginal smell.

10. Use according to claim 9 as a sexual stimulant or on a fetish article.

11. Use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, and vanillin for the manufacture of a pharmaceutical composition for treatment of reduced fertility of males and/or females.

12. Use of at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, and vanillin for the manufacture of a contraceptive.

13. Use according to claim 12, wherein the vaginal odorant compound is selected from the group consisting of n-hexanal, n-heptanal and n-nonanal.

14. Article for vaginal use comprising at least one vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, and vanillin.

15. Article according to claim 14, wherein the article is selected from the group consisting of vaginal crème, vaginal suppository, vaginal irrigation, vaginal lotion, vaginal ointment, tampon, sanitary napkin, fetish article and cloth.

16. A vaginal odorant compound selected from the group consisting of hydrogen sulfide, methylpropanal, butan-2,3-dione (diacetyl), n-hexanal, n-heptanal, n-nonanal, 1-octen-3-one, 2-acetyl-1-pyrroline, 2-acetylpyridine, 2-acetylthiazoline, 2-acetyltetrahydropyridine, 2-acetylpyrazine, 3-methylthio propanal (methional), (Z)-non-2-enal, 3-isobutyl-2-methoxypyrazine, (E)-non-2-enal, phenylacetaldehyde, (E)-β-damascenone, β-damascone, β-ionone α-ionone, tr-(4,5)-epoxy-(E)-dec-2-enal, 4-hydroxy-2,5-dimethyl-3(2H)-furanone, 3-hydroxy-4,5-dimethyl-2(5H)-furanone (Sotolon), (Z)-6-γ-dodecenolactone, δ-dodecalactone, 3-methylindole (skatol), indole, 2-aminoacetophenone, phenylacetic acid, and vanillin as a medicament.
